# EUROPEAN PATENT APPLICATION

(11) **EP 1 752 284 A1**
(43) Date of publication of application: **14.02.2007**
(21) Application number: 04745616.5
(22) Date of filing: 31.05.2004
(51) Int. Cl.: B32B 5/18, A61F 13/42

(54) **HYDROPHILIC POROUS FILM AND MULTI-LAYERED FILM COMPRISING THE SAME**

(71) Applicant: Mitsui Chemicals, Inc., Tokyo 105-7117 (JP)
(72) Inventor: YANO, Shigeru, Mitsui Chemicals, Inc., Nagoya-shi, Aichi 457-8522 (JP); ICHIKAWA, Taro, Mitsui Hygiene Materials, 21140 Rayong (TH)
(74) Representative: Wytenburg, Wilhelmus Johannes
(86) International application number: PCT/JP2004/007843
(87) International publication number: WO 2005/115738

(57) **Abstract**

A film-like material of the present invention comprises a multi-layered film comprising a hydrophilic porous film containing a thermoplastic resin as a main component and having a colored part on one face thereof and, provided on the other face thereof, a layer for preventing permeation of water. Furthermore, the hydrophilic porous film is obtained by forming a thermoplastic resin composition into a film, and then stretching the film in at least a uniaxial direction, wherein the thermoplastic resin composition comprises 25 to 80 parts by mass of a thermoplastic resin and 75 to 20 parts by mass of a filler and further a hydrophilicity imparting agent in an amount of 0.1 to 10 parts by mass relative to 100 parts by mass, where the total amount of the thermoplastic resin and the filler is 100 parts by mass.

## Description

### TECHNICAL FIELD

The present invention relates to a hydrophilic porous film and a multi-layered film comprising the film. More specifically, the invention relates to a multi-layered film which is usually white-colored before it is soaked in water (in a dry condition) and becomes transparent after it is soaked in water (in a wet condition), so that a pattern printed on the surface of the hydrophilic porous film can be seen from the backside thereof.

### BACKGROUND ART

In recent years, a porous film has increasingly been used as a back sheet of a paper diaper, which is effective in preventing urine leakage and diaper rash. Since there are innumerable minute voids formed inside the porous film, light is reflected in a diffused manner at an internal interface thereof, the porous film exhibits an opaque white color. For this reason, when the porous film is used as a back sheet of a paper diaper, the inside of the diaper is hardly visible from the outside. Since a color as light as urine is particularly difficult to detect, it is difficult to visually determine from the outside whether the wearer of a diaper has urinated or not. There is thus a need to actually take off the diaper to determine the condition thereof by hand, which is inconvenient.

In Japanese Utility Model Application Laid-Open (JP-U) No. 3-58416 or Japanese Patent Application Laid-Open (JP-A) No. 9-299401, materials in which white fine particles that become transparent upon absorbing water are fixed to a hydrophilic substrate of paper or the like, along with a resin binder, have been disclosed. When a printed pattern provided on the back surface of the substrate, and when the white fine particles on the surface and the substrate become transparent when they are soaked in water, the printed pattern becomes visible. This mechanism utilizes a physical change in the white fine particles having a refractive index close to that of water, wherein diffused reflection of light on the surface of the particles is reduced to increase transparency when the particles are soaked in water.

However, paper that is hard to the touch, such as is commonly used as a substrate, is not desirably used for purposes such as a diaper, where the material directly touches the skin. Furthermore, since production cost is high, it is not suitable for disposable usage such as for a paper diaper. Further, for the reason as described above, there is also a problem in that the area for display cannot be enlarged, and visibility is insufficient.

Thus, the present invention intends to provide a film-like material which is white before it is soaked in water (in the dry condition) and becomes transparent upon being soaked in water (in the wet condition), having improved flexibility compared to conventional materials, and having a colored part thereon. Further, the present invention intends to provide a film-like material which can be obtained at low cost as compared to conventional materials.

### DISCLOSURE OF THE INVENTION

The present inventors have conducted an extensive study and have found that the above object can be achieved by providing a hydrophilic porous film having a partially colored pattern on one face thereof and, provided on the other face thereof, a layer for preventing permeation of water. Thus, the present invention has been completed.

That is, the film-like material of the present invention comprises a multi-layered film including a hydrophilic porous film containing a thermoplastic resin as a main component, the hydrophilic porous film having a colored part on one face thereof, and a layer for preventing permeation of water on the other face thereof.

Further, the hydrophilic porous film of the present invention is obtained by forming a thermoplastic resin composition into a film and stretching the formed film at least in a uniaxial direction, the hydrophilic porous film containing 25 to 80 parts by mass of a thermoplastic resin and 75 to 20 parts by mass of a filler and further a hydrophilicity imparting agent in an amount of 0.1 to 10 parts by mass, where the total amount of the thermoplastic resin and the filler is 100 parts by mass.

### BRIEF DESCRIPTION OF THE DRAWINGS

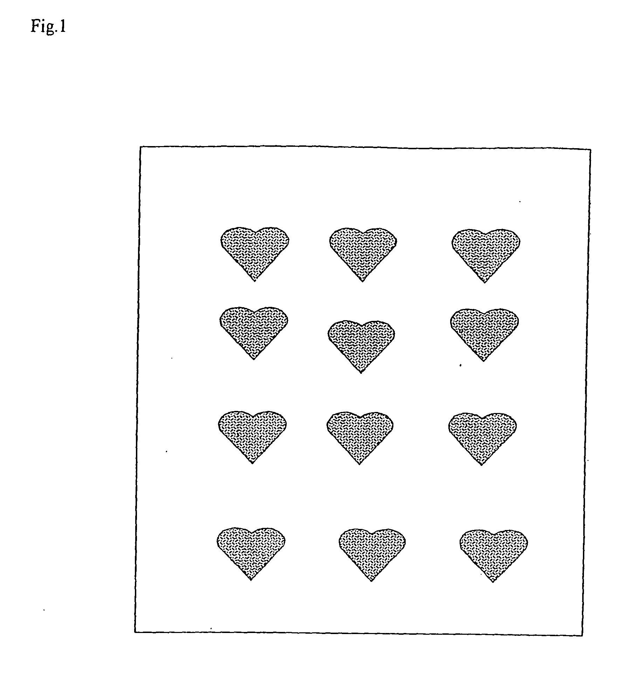

Fig. 1 is a schematic view of printed pattern used in Examples of the present invention.

### BEST MODE FOR CARRYING OUT THE INVENTION

The present invention will be described in more detail below.

The multi-layered film of the present invention is a multi-layered film comprising a hydrophilic porous film containing a thermoplastic resin as a main component, wherein the hydrophilic porous film has a colored part on one surface thereof, and a layer for preventing permeation of water on another surface.

Examples of the thermoplastic resin include a polyester resin, a polyamide resin, a polyolefin resin and the like; examples of the polyester resin include polyethylene terephthalate, polybutylene terephthalate and the like; and examples of the polyamide resin include nylon 6, nylon 66 and the like. Of these, a polyolefin resin is preferable from the viewpoint of excellent flexibility.

The polyolefin resin to be used for the present invention preferably mainly contains a polymer and a copolymer of olefins such as ethylene, propylene, butene, pentene, hexene, vinyl acetate and the like. Concrete examples thereof include polyethylene type resins such as low density polyethylene, linear low density polyethylene (ethylene-α-olefin copolymer), medium density polyethylene, high density polyethylene and the like; polypropylene type resins such as polypropylene, an ethylene-propylene copolymer and the like; poly4-methylpentene, polybutene, an ethylene-vinyl acetate copolymer, an ethylene-butene copolymer, and a mixture thereof. These polyolefin resins may be a resin prepared by using a Ziegler catalyst or may be a resin prepared by using a single site catalyst such as a metallocene catalyst.

Of these, a polyethylene type resin is more preferably used, and a linear low density polyethylene resin, i.e., an ethylene-α-olefin copolymer, and a low density polyethylene are most preferably used. The melt index of the polyolefin resin is preferably from about 0.1 to 30 g/10 min and more preferably from about 0.5 to 10 g/10 min in consideration of moldability, stretchability and the like of the film. The density of the polyolefin resin is preferably from 0.910 to 0.940 g/cm³ in consideration of flexibility of the film.

The hydrophilic porous film to be used for the multi-layered film of the present invention is preferably obtained by forming a film from a thermoplastic resin composition comprising 25 to 80 parts by mass of a thermoplastic resin and 75 to 20 parts by mass of a filler, and then stretching the film.

As the filler, an inorganic filler or an organic filler can be used. Examples of the inorganic filler include calcium carbonate, barium sulfate, calcium sulfate, barium carbonate, magnesium hydroxide, aluminum hydroxide, zinc oxide, magnesium oxide, titanium oxide, silica, talc, glass beads and the like. Of these, barium sulfate and calcium carbonate are preferred. Considering inexpensiveness, calcium carbonate is more preferable. As the organic filler, preferably used are resin beads of polystyrene, poly (methyl methacrylate), phenolic resin and the like.

The composition ratio of the thermoplastic resin and the filler exerts influence on moldability and stretchability of the film, light transmittance of the obtained film in dry and wet conditions, and the like. The amount of the filler being too small is not preferable since the number of minute voids obtained by separation of the thermoplastic resin and the filler at the interface thereof is reduced and the light transmittance in the dry condition is increased. On the other hand, the amount of the filler being too great is not preferable since defective molding at the time of molding a film may occur or stretching may not be sufficiently carried out due to lowered stretchability. In view of the foregoing, the composition ratio of the thermoplastic resin and the filler is preferably from 25 to 80 weight % of the thermoplastic resin and from 75 to 20 weight % of the filler. It is more preferably from 30 to 70 weight % of the thermoplastic resin and 70 to 30 weight % of the filler. The average particle diameter of the filler is preferably 20 µm or less, more preferably 10 µm or less and most preferably from 0.5 to 5 µm.

The filler may be subjected to a surface treatment for improving dispersability with a thermoplastic resin. As the surface treatment agent, those capable of making the surface of the filler hydrophobic by covering the surface of the filler are preferred. For example, higher fatty acids such as stearic acid, lauric acid and the like, or metal salts thereof can be mentioned.

The hydrophilic porous film to be used for the multi-layered film of the present invention is preferably obtained by forming a film from a thermoplastic resin composition comprising 25 to 80 parts by mass of a thermoplastic resin and 75 to 20 parts by mass of a filler, and further a hydrophilicity imparting agent in an amount of 0.1 to 10 parts by mass, where the total amount of the thermoplastic resin and the filler is 100 parts by mass, and then stretching the film.

The hydrophilic porous film is characterized by containing a specified amount of hydrophilicity imparting agent in a resin composition containing a thermoplastic resin and a filler. The amount of the hydrophilicity imparting agent exerts influence on the light transmittance of the film in the wet condition.

The amount of the hydrophilicity imparting agent added being too small is not preferable since permeation of water into voids inside the film becomes difficult when it is soaked in water, and the light transmittance in the wet condition is lowered. On the other hand, the amount of the hydrophilicity imparting agent added being too great is not preferable since bleed-out of the hydrophilicity imparting agent may occur during storage or extrusion performance may be unfavorable, although the light transmittance in the wet condition is increased. In view of the foregoing, the amount of the hydrophilicity imparting agent added is preferably from 0.1 to 10 parts by weight and more preferably from 0.5 to 5 parts by weight, where the total amount of the resin composition containing the thermoplastic resin and the filler is 100 parts by weight.

Examples of the hydrophilicity imparting agent include nonionic surface active agents, anionic surface active agents, cationic surface active agents and the like. Concrete examples of the nonionic surface active agent include polyoxyethylene alkyl ether, glycerin fatty acid ester, polyethylene glycol fatty acid ester, polyoxyethylene fatty acid ester and sorbitan fatty acid ester; examples of the anionic surface active agent include alkylsulfate ester salt and polyoxyethylene alkylether sulfate ester salt; and examples of the cationic surface active agent include alkylamine salt and the like. Of these, a nonionic surface active agent is preferred because it is hardly affected by other ionic contents. Furthermore, at least one compound selected from fatty acid ester compounds is preferred because hydrophilicity thereof is easy to control.

As the fatty acid ester, an ester of polyhydric alcohol with fatty acid can be cited. Examples of the polyhydric alcohol include glycerin, polyglycerin, sorbitan, ethylene glycol, polyethylene glycol, propylene glycol, polypropylene glycol and the like. Examples of the fatty acid include coconut fatty acid, tallow fatty acid, caprylic acid, lauric acid, myristic acid, palmitic acid, stearic acid, behenic acid, oleic acid and the like. Of the hydrophilicity imparting agents comprising the aforementioned fatty acid ester, polyethylene glycol fatty acid ester is the most preferably used in consideration of bleed-out of the hydrophilicity imparting agent during storage, migration of the hydrophilicity imparting agent during use and moldability of the film.

An average molecular weight of polyethylene glycol constituting the polyethylene glycol fatty acid ester exerts influence on bleed-out of the hydrophilicity imparting agent during storage, migration of the hydrophilicity imparting agent during use or moldability of the film. The average molecular weight of polyethylene glycol being too low is not preferable since the hydrophilicity imparting agent easily bleeds out during storage and denatures over time, and, the hydrophilicity imparting agent easily migrates during use, in particular, the hydrophilicity imparting agent is washed away due to moisture and diffused to the periphery. In addition, it is not preferable because generation of smoke becomes great at the time of melt extrusion molding.

On the other hand, the average molecular weight of polyethylene glycol being too high is not preferable since the extrusion amount is decreased, thus degrading the film productivity. For this reason, the average molecular weight of polyethylene glycol constituting the hydrophilicity imparting agent is preferably from 150 to 750 and more preferably from 300 to 500. In the present invention, the average molecular weight can be measured by a usual method using gel permeation chromatography.

The number of carbon atoms of a hydrocarbon group of fatty acid constituting polyethylene glycol fatty acid ester also exerts influence on bleed-out of the hydrophilicity imparting agent during storage, migration of the hydrophilicity imparting agent during use or moldability of the film. The number of carbon atoms of fatty acid being too small is not preferable since the hydrophilicity imparting agent easily bleeds out during storage and denatures over time, and the hydrophilicity imparting agent easily migrates during use, in particular, the hydrophilicity imparting agent is washed away due to moisture and diffused to the periphery. Furthermore, it is not preferable because the extrusion performance is reduced. The number of carbon atoms of fatty acid being too great is not preferable since hydrophilicity is lowered so that the transmittance in the wet condition of the present invention cannot be enhanced. For that reason, the number of carbon atoms of fatty acid is preferably from 12 to 16 and more preferably from 12 to 14.

Concrete examples of the above fatty acid ester include TB-202 or TB1259 (product name, a product of Matsumoto Yushi-Seiyaku Co., Ltd.), Rikemal O-71-DE (product name, a product of Riken Vitamin Co., Ltd.) and the like. However, fatty acid esters are not limited thereto as far as they satisfy the above contents.

In the hydrophilic porous film to be used for the multi-layered film of the present invention, other additives may be added so long as they do not impair the object of the present invention, and examples of the additives include a stretching auxiliary agent, a stabilizer, an antioxidant, a colorant, an ultraviolet light absorber, a dispersant and the like.

Next, a method for preparing the multi-layered film of the present invention will be exemplified. First, in order to prepare a hydrophilic porous film, for example, the aforementioned thermoplastic resin, the filler, the hydrophilicity imparting agent, and if necessary, other additives are mixed by using a Henschel mixer, a super mixer, a tumbler type mixer or the like, and then kneaded and palletized by using a single screw extruder or a twin screw extruder. Next, the pellets are melted and molded into a film at a temperature of a melting point or more of the thermoplastic resin, and preferably at a temperature higher than the melting point by +20 °C or more, but lower than the decomposition temperature of the thermoplastic resin, by using a known molding machine such as an extrusion molding machine equipped with a T-die or the like, an inflation molding machine equipped with a circular die, and the like. Depending on the case, the composition can be directly formed into a film by a molding machine without pelletization.

The thus formed film can be stretched at least in a uniaxial direction at a temperature range of from room temperature to the softening point of the resin (a value measured by a method specified in JIS K-6760) according to a known method such as a roll method, a tenter method, a gear stretch method or the like to cause separation of the thermoplastic resin and the filler from each other at the interface thereof, whereby a porous film can be prepared. The stretching may be carried out through a single step or plural steps. The stretching factor is related to film breakage at the time of stretching, as well as light transmission of the obtained film in the dry condition, thus neither of the ratio being excessively high nor excessively low are preferable. In view of this point, the stretching factor is preferably from 1.2 to 6 times and more preferably from 1.4 to 5 times at least in a uniaxial direction. In the case that biaxial stretching is carried out, there is a method in which the first stretching is performed in a machine direction or a direction at a right angle thereto, and the second stretching is performed in a direction at a right angle to the direction in which the first stretching has been performed. There is also another method in which stretching is performed simultaneously in a biaxial manner, in the machine direction and the direction at a right angle thereto. Either of these methods can be applied to the present invention.

Meanwhile, after stretching, a heat setting treatment may be carried out in order to stabilize the shape of obtained voids, if necessary. As the heat setting treatment, for example, there is a method comprising carrying out a heat treatment at a temperature of from the softening point of the resin or higher, but less than the melting point of the resin for a period of 0.1 to 100 seconds. Further, a corona treatment may be carried out for facilitating coloring of the film by printing or the like, and facilitating water permeation into the film.

The multi-layered film of the present invention is partially colored on one face thereof, for visibility. Coloring processes are not particularly limited as far as the object of the present invention is achieved. However, preferably used is a printing process because of a small increase in weight due to coloration. By performing printing on one surface of the film, an effect can be obtained, wherein the printed pattern is not visible from the other face of the film in a dry condition, but is visible through in a wet condition. The method of printing is not particularly limited and examples thereof include a flexographic printing, a gravure printing and the like, and the ink to be used is not limited and examples thereof include solvent-based ink, water-based ink and the like. The color of printing is not particularly limited and may either single colored or multi colored. The printed pattern is not particularly limited, but in a solid printed part, water permeation into the voids inside the film tends to be difficult, and tends to take longer time to become transparent in the wet condition. On the contrary, a dot printed part becomes transparent in a short period of time because water permeates easily into the voids inside the film, even from the printed part. It is preferable to adjust the part printed in a solid or dotted manner as appropriate according to usage. It is also possible to impart different properties against water to the front and back surfaces, respectively.

Furthermore, in order to make the whole surface of the film easy to be in the wet condition, it is also effective to provide a layer comprising a high polymer absorbent such as a high absorbing polymer and the like on the printed colored part. The layer may either be the entire surface of the film or a part thereof. The high polymer absorbent layer can be provided on the printed pattern according to a method such as performing further printing by a printing machine. In that case, the layer may be applied either entirely or partially in a dotted manner. The thickness of the layer is not particularly limited, but when the layer is extremely thin, it fails to absorb water effectively, while when the layer is extremely thick, flexibility or inexpensiveness is unfavorable. In view of these points, the thickness of the layer is usually from about 0.1 to about 50 µm.

The multi-layered film of the present invention preferably has a layer for preventing permeation of water provided on the opposite face to the face which is colored for visibility. By providing the layer for preventing permeation of water, when the film is used for visualization of urine and the like, elution of a hydrophilicity imparting agent in the film by urine can be prevented. When the hydrophilicity imparting agent is eluted by urine, a moisture permeable porous film, which is commonly used as a leak proof film for a diaper or the like, may come in contact with urine, making the leak proof film hydrophilic to cause leakage of urine or the like.

Furthermore, by providing the layer for preventing permeation of water, moisture such as urine that has permeated into the porous film can be prevented from drying. When moisture disappears from the porous film as being dried, light transmittance turns back to that in the dry condition, so the printed pattern becomes invisible.

The layer for preventing permeation of water may be formed into a multi-layered film by a multi-layer extrusion process. For example, a method can be used, wherein a film comprising a thermoplastic resin layer containing a filler and a hydrophilicity imparting agent, and a film comprising a thermoplastic resin layer not containing a filler are melted and molded into a film by using a multi-layer extrusion film-forming machine equipped with two extrusion molding machines and a T-die, and then the film was uniaxially stretched in the machine direction between a preheat roll and a stretching roll. Further, a water proof material may be coated on the film. For example, a method of coating an acrylic resin using a roll coater and the like can be cited.

In addition, it is preferable to provide the layer for preventing permeation of water by performing printing on the whole surface of the porous film with a transparent printing ink including no colorant and drying the ink, because of easiness and inexpensiveness. Printing methods are not particularly limited and examples thereof include flexographic printing, gravure printing and the like. The ink is also not limited and examples thereof include a solvent-based ink, a water-based ink and the like. However, the solvent-based ink, being superior in properties of preventing water permeation, is preferable. The thickness of the layer for preventing permeation of water is not particularly limited, but when the layer is extremely thin, it fails to prevent permeation of water effectively, while when the layer is extremely thick, flexibility or inexpensiveness is unfavorable. In view of these points, the thickness is usually from about 0.1 to about 50 µm.

The thickness of the multi-layered film of the present invention is not particularly limited. However, the film being extremely thick is not preferable since not only light transmittance in the wet condition is lowered, but also flexibility or inexpensiveness is lowered. On the other hand, the film being extremely thin is not preferable since breakage may occur upon molding. In view of these points, the thickness is usually from about 5 to about 100 µm, preferably from 7 to 70 µm and more preferably from 8 to 50 µm.

It is preferable that the multi-layered film of the present invention prepared in a manner as described above has the total light transmittance in the range of less than 50%, in a dry condition, and the total light transmittance in the range of 50% or more, in a wet condition. It is not desirable to use a film having a light transmittance in the dry condition of 50% or more for detecting moisture, since difference between the light transmittance in the dry condition and the light transmittance in the wet condition becomes small, thus it is difficult to perceive the difference that appears when the film gets wet. It is also not desirable to use a film having a light transmittance in the wet condition of less than 50% for detecting moisture, since it is difficult to make the printed pattern visible from the back surface. The total light transmittance in the dry condition is more preferably less than 45%, while the total light transmittance in the wet condition is 55% or more. Further, the difference between the total light transmittance in the dry condition and the total light transmittance in the wet condition is preferably 15% or more, and more preferably 20% or more.

The multi-layered film of the present invention has a suitable flexibility, with a rigidity of 80 mm or less. Therefore, the multi-layered film can be suitably used, for example, for a use application requiring flexibility such as a film for detecting urine in a disposable diaper. The lower limit of the rigidity is not particularly specified, but it is usually about 10 mm.

The multi-layered film having such characteristics can be suitably used as a film for detecting moisture because of the low total light transmittance in the dry condition and the high total light transmittance in the wet condition. Further, since the multi-layered film has a high degree of flexibility and is inexpensive, it is suitable for a film for detecting urine in a disposable paper diaper and the like.

### EXAMPLES

Examples are shown below for explaining the present invention now more specifically. Incidentally, the present invention is not restricted to these Examples.

Melt index (hereinafter referred to as MI), the total light transmittance in the dry condition, the total light transmittance in the wet condition, film thickness, rigidity, migration of a hydrophilicity imparting agent and drought resistance, shown in Examples, are values measured by the following methods.
(1) Melt index (g/10 min)
   The melt index was measured under the conditions of a temperature of 190 degree centigrade and a load of 2160 g in accordance with the method specified in ASTM D-1238-57T (E).
(2) Total light transmission (%) in the dry condition
   The total light transmission in the dry condition was measured by using a haze meter (a product of Nippon Denshoku Kogyo Co., Ltd., type: NDH-300A) based on the method specified in JIS K-7105. Ten samples were prepared, wherein the size of each sample was 50 mm in a machine direction (hereinafter referred to as MD direction) and 100 mm in a direction at a right angle to the machine direction (hereinafter referred to as TD direction) of the multi-layered film. Measurement was conducted at two points outside the printed pattern area for each sample, thus 20 points for the ten samples in total, then the average value thereof was calculated.
(3) Total light transmission (%) in the wet condition
   Ten samples were prepared in the same manner as in (2) above, and after dipping in pure water for 1 minute, drops of water were wiped off from the surface. Measurement was conducted at two points for each sample by using a haze meter. The above operation was repeated for each sample to measure 20 points from ten samples in total. The average value thereof was calculated.
(4) Film thickness
   Ten sheets of samples (MD direction: 10 cm, CD direction: 10 cm) were collected from the multi-layered film. The thickness of each sample was measured at 5 measuring points, thus 50 points in ten samples in total, by using a thickness measuring device (a product of Peacock Co., Ltd., UPRIGHT DIAL GUAGE NO. 25). The average thickness thereof was calculated to be regarded as the film thickness.
(5) Rigidity (mm)
   The rigidity was measured in accordance with the method specified in JIS-L1096 (cantilever method). The sample used for the measurement was prepared by winding a film having a width of 200 mm and a length of 300 mm around a carpenter's square having a width of 25 mm, and after the square was taken out, pressing the obtained flat-shaped roll (a width of 25 mm and a length of 300 mm) by a roller of 1 kg weight by one reciprocation stroke.
(6) Migration of hydrophilicity imparting agent
   The porous film of 200 square-mm prepared according to Example 1 except that a hydrophilicity imparting agent was not used, the multi-layered film of the present invention of 200 square-mm, and two sheets of filter paper of 100 square-mm were piled up in this order, and 5 ml of water tinted with an ink was dropped on the filter paper. Then, a weight of 100 square-mm and 10 kg was put on the filter paper, and after 5 minutes, the bottom surface (the back surface of the porous film without a hydrophilicity imparting agent) was visually observed as to whether permeation of ink occur within the range of 100 square-mm and evaluated in accordance with the following criteria.
   ○: No permeation was observed
   Δ: Permeation was observed within the half of the 100 square-mm area
   × : Permeation was observed in the whole 100 square-mm area, exceeding the half area thereof.
(7) Drought resistance
   A transparent acryl plate was put on a black cardboard of A4 size, and 0.1 cc of water was dropped thereon. The multi-layered film of 3 cm × 4 cm of the present invention was put thereon quietly with a printed face underneath After 1 minute, 15 minutes and 30 minutes, appearance of the printed pattern was visually observed. Further, for the purpose of comparison, the multi-layered film in the dry condition was also observed and evaluated in accordance with the following criteria. The temperature was 23 degree centigrade and the relative humidity was 50%.
   ○: Distinctly observed
   Δ: Faintly observed
   ×: Almost nothing was observed
(8) Moldability
   Using an extrusion molding machine equipped with a T-die, a film was melted and formed into a film at 240 degree centigrade, and then the film was uniaxially stretched in the machine direction between a preheat roll and a stretching roll heated at 70 degree centigrade by a stretching factor of 4.0 times and at a line speed of 20 m/min after stretching. At that time, state of the stretching was observed and evaluated in accordance with the following criteria.
   ○: Molding can be performed in a stable manner without film breakage or a drastic reduction in the amount of extrusion
   × : Molding cannot be performed in a stable manner because of film breakage or a drastic reduction in the amount of extrusion

### Example 1

38 parts by weight of linear low density polyethylene LLDPE1 (a product of Mitsui Chemicals, Inc., product name: Ultzex 2021 L, density: 0.920 g/cm³, melt index (MI): 2.1 g/10 min), 2 parts by weight of branched low density polyethylene LDPE (a product of Mitsui Chemicals, Inc., product name: Mirason 27, density: 0.918 g/cm³, MI: 2.0 g/10 min), 60 parts by weight of calcium carbonate CaCO₃ (a product of Dowa Calfin Co., Ltd, product name: SST-40, average particle diameter: 1.1 µm) as a filler, and 3 parts by weight of a hydrophilicity imparting agent A (a product of Matsumoto Yushi-Seiyaku Co., Ltd., product name: TB-202, polyethylene glycol fatty acid ester) using a tumbler mixer, and then they were uniformly kneaded at 200 degree centigrade by using a tandem type extruder, and processed into the form of pellets. The pellets were melted and formed into a film at 240 degree centigrade using an extrusion molding machine equipped with a T-die, and then the film was uniaxially stretched in the machine direction between a preheat roll and a stretching roll heated at 70 degree centigrade, where the stretching factor was 4.0 times, to obtain a hydrophilic porous film having a thickness of 25 µm. On one face of the film, coloring was performed by printing a pattern (half-tone printing, 50 line/inch, 50%) as shown in Fig. 1 by means of a central impression flexographic press machine, with an ink prepared by adjusting an ink, i.e., Hydric SK293C Blue (H) manufactured by Dainichiseika Color & Chemicals Mfg. Co., Ltd., to have a viscosity of 20 seconds by adding a solvent, i.e., Hydric SK No. 2 manufactured by Dainichiseika Color & Chemicals Mfg. Co., Ltd., with a Zahn cup No. 3. The total light transmission in a dry condition, the total light transmission in a wet condition, rigidity, migration of a hydrophilicity imparting agent, drought resistance and moldability of the obtained porous film were measured and evaluated according to the aforemenetioned method. The obtained results are shown in Table 1.

### Example 2

On the opposite face to the printed face of the film prepared in Example 1, an ink component (medium) including no colorant was printed (applied) on the entire face of the film using an entirely anastatic printing plate in a central impression flexographic press, wherein the ink was prepared by adjusting an ink composition including no colorant, i.e., FPOT-NWF (so-called new version of Medium MT) manufactured by Osaka Printing Ink Mfg. Co., Ltd. to have a viscosity of 20 seconds by adding a No. 3 solvent of an ink solvent manufactured by Osaka Printing Ink Mfg. Co., Ltd., using a Zahn cup No. 3. The evaluation results of the obtained multi-layered film are shown in Table 1.

### Examples 3 to 8, Comparative Examples 1 to 2

A multi-layered film was prepared in the same manner as in Example 2 except that the combination ratio (unit: parts by weight) of the linear low density polyethylene, the filler and the hydrophilicity imparting agent, as well as the stretching factor and film thickness were changed as shown in Tables 1 and 2. The characteristics of the obtained film were measured and evaluated in the same manner as in Example 1. The results thereof are shown in Tables 1 and 2.

### Example 9

On the printed face of the film prepared in Example 2, a water absorbing polymer type coating agent was printed (applied) on the entire face of the film by means of a central impression flexographic press machine and using an entirely anastatic printing plate, wherein the coating agent was a mixture of EXP15078 (water absorbing medium) and EXP15078 (curing agent), both are produced by Osaka Printing Ink Mfg. Co., Ltd., at the mixing ratio of 100:1. The obtained results are shown in Table 1.

### Example 10

A multi-layered film was obtained in the same manner as in Example 2 except that the printed face of the film obtained in Example 1 was subjected to a corona treatment at 1 kV using a corona treatment device, before printing. The obtained results are shown in Table 1.

**[Table 1]**

| Example | | Unit | Example 1 | Example 2 | Example 3 | Example 4 | Example 5 | Example 6 | Example 7 | Example 8 | Example 9 | Example 10 |
|---|---|---|---|---|---|---|---|---|---|---|---|---|
| Resin | | Parts by | LLDPE1 | LLDPE1 | LLDPE1 | LLDPE1 | LLDPE1 | LLDPE1 | LLDPE1 | LLDPE1 | LLDPE1 | LLDPE1 |
| | | weight | 38 | 38 | 47.5 | 28.5 | 38 | 38 | 38 | 38 | 38 | 38 |
| Resin | | Parts by | LDPE | LDPE 2 | LDPE | LDPE | LDPE | LDPE | LDPE | LDPE | LDPE | LDPE |
| | | weight | 2 | 2 | 2.5 | 1.5 | 2 | 2 | 2 | 2 | 2 | 2 |
| Filler | | Parts by | CaCO₃ | CaCO₃ | CaCO₃ | CaCO₃ | CaCO₃ | CaCO₃ | CaCO₃ | CaCO₃ | CaCO₃ | CaCO₃ |
| | | weight | 60 | 60 | 50 | 70 | 60 | 60 | 60 | 60 | 60 | 60 |
| Hydrophilic agent | | Parts by | A | A | A | A | A | A | A | A | A | A |
| | | weight | 3 | 3 | 3 | 3 | 3 | 3 | 3 | 3 | 3 | 3 |
| Stretching factor | | Times | 4.0 | 4.0 | 4.0 | 4.0 | 2.0 | 3.0 | 4.0 | 4.0 | 4.0 | 4.0 |
| Layer for preventing permeation of water | | | No | Yes | Yes | Yes | Yes | Yes | Yes | Yes | Yes | Yes |
| Thickness | | µm | 25 | 25 | 25 | 25 | 25 | 25 | 20 | 45 | 25 | 25 |
| Rigidity | MD | mm | 35 | 35 | 42 | 33 | 30 | 32 | 32 | 43 | 39 | 35 |
| Total light transmission | Dry | % | 27 | 27 | 38 | 23 | 46 | 36 | 30 | 18 | 27 | 27 |
| | Wet | % | 62 | 62 | 60 | 63 | 72 | 63 | 63 | 52 | 63 | 62 |
| Migration of hydrophilic agent | | | Δ | ○ | ○ | ○ | ○ | ○ | ○ | ○ | ○ | ○ |
| Drought resistance | | Dry condition | × | × | × | × | Δ | × | × | × | × | × |
| | | After 1min | ○ | ○ | ○ | ○ | ○ | ○ | ○ | Δ | ○ | ○ |
| | | After 15 min | ○ | ○ | ○ | ○ | ○ | ○ | ○ | Δ | ○ | ○ |
| | | After 30 min | × | ○ | ○ | ○ | ○ | ○ | ○ | Δ | ○ | ○ |
| Moldability | | | ○ | ○ | ○ | ○ | ○ | ○ | ○ | ○ | ○ | ○ |

**[Table 2]**

| Example | Unit | Comparative Example 1 | Comparative Example 2 |
|---|---|---|---|
| Resin | Parts by weight | LLDPE1 | LLDPE1 |
| | | 19 | 38 |
| Resin | Parts by weight | LDPE | LDPE |
| | | 1 | 2 |
| Filler | Parts by weight | CaCO₃ | CaCO₃ |
| | | 80 | 60 |
| Hydrophilic agent | Parts by weight | A | A |
| | | 3 | 15 |
| Moldability | | × (film broken) | × (decrease in the amount of extrusion) |

### INDUSTRIAL APPLICABILITY

The multi-layered film of the present invention has an improved flexibility, is much cheaper, and has a function of detecting moisture such as urine or the like equal to conventional products. For that reason, the film can be suitably applied in the fields of sanitary materials, medical materials, clothing materials, building materials, packing materials and the like, and can be suitably used, in particular, as a film for detecting urine in an absorbent item such as a disposable diaper and the like.

## Claims

1. A multi-layered film comprising a hydrophilic porous film containing a thermoplastic resin as a main component, wherein the hydrophilic porous film has a colored part on one surface thereof, and a layer for preventing permeation of water on another surface.

2. The multi-layered film of claim 1, wherein the hydrophilic porous film is obtained by forming a thermoplastic resin composition into a film and then stretching the formed film, the thermoplastic resin composition comprising 25 to 80 parts by mass of a thermoplastic resin and 75 to 20 parts by mass of a filler.

3. The multi-layered film of claim 2, wherein the thermoplastic resin composition further comprises a hydrophilicity imparting agent in an amount of 0.1 to 10 parts by mass, where the total amount of 25 to 80 parts by mass of the thermoplastic resin and 75 to 20 parts by mass of a filler is 100 parts by mass.

4. The multi-layered film of claim 1, wherein the layer for preventing permeation of water is obtained by coating the hydrophilic porous film with a printing ink containing no colorant, and drying the ink.

5. The multi-layered film of claim 1 having a layer comprising a high polymer absorbent on the colored part.

6. A film for detecting moisture comprising the multi-layered film of any one of claims 1 to 5.

7. A hydrophilic porous film obtained by forming a thermoplastic resin composition into a film, and then stretching the film in at least one axial direction, the thermoplastic resin composition comprising 25 to 80 parts by mass of a thermoplastic resin and 75 to 20 parts by mass of a filler, and further comprising a hydrophilicity imparting agent in an amount of 0.1 to 10 parts by mass, where the total amount of the thermoplastic resin and the filler is 100 parts by mass.
